# EUROPEAN PATENT SPECIFICATION

(11) **EP 3 104 767 B1**
(45) Date of publication and mention of the grant of the patent: **22.03.2023**
(21) Application number: 15703908.2
(22) Date of filing: 29.01.2015
(51) Int. Cl.: A61B 5/00, A61B 5/024, A61B 5/0205, A61B 5/1455

(54) **DEVICE, SYSTEM AND METHOD FOR DETERMINING VITAL SIGNS OF A SUBJECT BASED ON REFLECTED AND TRANSMITTED LIGHT**
VORRICHTUNG, SYSTEM UND VERFAHREN ZUR BESTIMMUNG VON LEBENSZEICHEN AUS REFLEKTIERTEM UND ÜBERTRAGENEM LICHT
DISPOSITIF, SYSTÈME ET PROCÉDÉ PERMETTANT DE DÉTERMINER DES SIGNES VITAUX CHEZ UN SUJET SUR LA BASE DE LA LUMIÈRE RÉFLÉCHIE ET TRANSMISE

(30) Priority: 12.02.2014 EP 14154890; 12.02.2014 US 201461938919 P
(43) Date of publication of application: 21.12.2016
(73) Proprietor: Koninklijke Philips N.V., 5656 AG Eindhoven (NL)
(72) Inventor: KIRENKO, Ihor Olehovych, 5656 AE Eindhoven (NL)
(74) Representative: Philips Intellectual Property & Standards
(86) International application number: PCT/EP2015/051747
(87) International publication number: WO 2015/121070

(56) References cited:
- WO-A1-2013/166341
- WO-A1-2013/186696
- WO-A2-2013/027141
- US-A1- 2008 194 906
- US-A1- 2009 299 154
- US-A1- 2014 031 696

## Description

### FIELD OF THE INVENTION

The present invention relates to a system and method for determining vital signs of a subject, such as a person or animal.

### BACKGROUND OF THE INVENTION

Vital signs of a person, for example the heart rate (HR), the respiration rate (RR) or the arterial blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heartbeat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation (also called SpO2) of a subject are attached to the skin of the subject, for instance to a fingertip, earlobe or forehead. Therefore, they are referred to as 'contact' PPG devices. A typical pulse oximeter comprises a red LED and an infrared LED as light sources and one photodiode for detecting light that has been transmitted through patient tissue. Commercially available pulse oximeters quickly switch between measurements at a red and an infrared wavelength and thereby measure the transmittance of the same area or volume of tissue at two different wavelengths. This is referred to as time-division-multiplexing. The transmittance over time at each wavelength gives the PPG waveforms for red and infrared wavelengths. Although contact PPG is regarded as a basically non-invasive technique, contact PPG measurement is often experienced as being unpleasant and obtrusive, since the pulse oximeter is directly attached to the subject and any cables limit the freedom to move and might hinder a workflow.

Fast and reliable detection and analysis of a pulse signal and oxygen saturation level (SP02) is one of the most important activities in many healthcare applications, which becomes crucial if a patient is in a critical condition. In those situations, pulsatility of a heartbeat signal is very weak, and therefore, the measurement is vulnerable to any sort of artifacts.

Modern photoplethysmography sensors do not always provide fast and reliable measurement in critical situations. For instance, contact finger pulse oximeters (based on transmissive PPG) are vulnerable to motion of a hand, and fails in case of centralization of a patient due to lower blood volumes on body peripherals. Contact forehead pulse oximeter sensors (using a reflective PPG measurement mode) are supposed to be more robust to a centralization effect. However, the accuracy, robustness and responsiveness of a forehead sensor depends heavily on correct positioning of a sensor on a forehead and proper pressure applied to a skin (too tight application of a sensor might reduce a local blood pulsatility, too loose application might lead to non-reliable measurements due to motion artifacts and/or venous pulsatility).

Recently, non-contact, remote PPG (rPPG) devices (also called camera rPPG device herein) for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications. However, remote PPG devices typically achieve a lower signal-to-noise ratio.

Verkruysse et al., "Remote plethysmographic imaging using ambient light", Optics Express, 16(26), 22 December 2008, pp. 21434-21445 demonstrates that photoplethysmographic signals can be measured remotely using ambient light and a conventional consumer level video camera, using red, green and blue colour channels.

Wieringa, et al., "Contactless Multiple Wavelength Photoplethysmographic Imaging: A First Step Toward "SpO2 Camera" Technology," Ann. Biomed. Eng. 33, 1034-1041 (2005), discloses a remote PPG system for contactless imaging of arterial oxygen saturation in tissue based upon the measurement of plethysmographic signals at different wavelengths. The system comprises a monochrome CMOS-camera and a light source with LEDs of three different wavelengths. The camera sequentially acquires three movies of the subject at the three different wavelengths. The pulse rate can be determined from a movie at a single wavelength, whereas at least two movies at different wavelengths are required for determining the oxygen saturation. The measurements are performed in a darkroom, using only one wavelength at a time.

Using PPG technology, vital signs can be measured, which are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or high required accuracy of the application, where an improved robustness and accuracy of the vital sign measurement devices and methods is required, particularly for the more critical healthcare applications.

US 2008/0194906 A1 discloses a non-intrusive physiological data measurement system and method, as well as an optically induced treatment system. The measurement system includes a monitoring mechanism that includes light emitter modules capable of emitting light at at least two wavelengths. The light emitted from the light emitter modules is transmitted through a subject and to a light receiving mechanism, such as an optical sensor. Physiological data is taken from the received light. The system also can ascertain movement of the subject by obtaining an initial outline of the subject and comparing that outline with a subsequently obtained outline. A therapeutic optic system includes a non-adhering light emitting mechanism for providing light at therapeutic wavelengths.

WO 2013/027141 A2 discloses a data administration system comprising a sensor unit for obtaining image data of at least a body part of a living being a vital sign extraction unit for extracting one or more vital signs of said living being from the obtained image data of said living being, a feature extraction unit for extracting one or more features of said living being from the obtained image data of said living being, an identification unit for determining the identity of said living being by use of said one or more extracted features of said living being, and a data association unit for associating the one or more extracted vital signs of said living being with the determined identity of said living being.

WO 2013/186696 A1 discloses measurement of vital signs such as a respiratory rate or a heart rate. In particular, a system for determining a vital sign of a subject, comprising an imaging unit for obtaining video data of the subject, a marker directly or indirectly attached to a body of the subject, wherein the marker comprises a graphical pattern, an image processing unit for detecting said marker in said video data, and an analysis unit adapted to extract a vital sign parameter related to the vital sign of the subject from said video data and to determine the vital sign from said vital sign parameter.

### SUMMARY OF THE INVENTION

It an object of the present invention to provide an improved system and method for determining vital signs of a subject having an increased signal-to-noise ratio and efficiency in reduction of artefacts caused by distortions, in particular by motion of the subject or disturbance from ambient light.

According to the present invention a system and method for determining vital signs of a subject are presented as defined in the claims.

Preferred embodiments of the invention are defined in the dependent claims. It shall be understood that the claimed method has similar and/or identical preferred embodiments as the claimed system and as defined in the dependent claims.

The present invention is based on the following findings. A classical embodiment of a vital signs camera (i.e. a device for determining vital signs of a subject) for remote PPG uses a reflective method of measurement of PPG signal, where an ambient or dedicated illumination is used. Although such reflective remote PPG technique is the most convenient, in cases of severe disturbance from ambient illumination, or if a pulsatility of a PPG signal is very low, SNR of the extracted signal will be weak and the measurements would be vulnerable to artifacts. The reason for this is the fact that pulsatility of a PPG signal measured in reflective mode is lower, than the one measured in a transmissive mode, due to a smaller penetration depth of a light in a reflective mode. Especially, pulsatility of a PPG signals measured in red and infrared color range using remote reflective PPG approach might be very low, thus reducing an accuracy of SPO2 measurement.

The present invention overcomes the limitations of contactless camera-based reflective PPG technique and classical contact sensors by combining the advantages of transmissive mode for PPG signal acquisition and contactless sensing using a common detector, such as a camera. The invention thus makes use of advantages of a large sensing area, achieved with (particular camera-based) measurements in reflective mode, and a relatively large SNR of a PPG signal extracted in a transmissive mode.

According to the present invention a single detector (e.g. image sensor or camera) measures reflected and transmitted light simultaneously, and PPG signals are extracted from both reflected and transmitted illumination. The wavelengths of transmitted and reflected light used for evaluation and derivation of vital signs are complementary (e.g. green and red wavelengths are used from the reflected light, IR wavelengths are used from the transmitted light). Thus, evaluated wavelengths in transmissive and reflective modes are different. One or more vital signs may then be obtained with higher accuracy and robustness from the original PPG signal or one or more combined PPG signals.

The transmitted and reflected light may be obtained from the same or from different regions of interest (ROIs). For instance, for HR measurement, an acquisition of reflected (e.g. at green wavelength) and transmitted (in IR range) PPG signals is possible and useful. For SpO2, it is preferred to know the calibration constants for various body locations used for reflective PPG. Thus, calibration constants used for SPO2 calculations for the case when both reflective and transmissive PPG come from the same ROI and for the case when they are measured from different locations would be different. Generally, however, the transmitted PPG signals and the reflective PPG signal may be obtained from different ROIs or from the same ROI.

Generally, the interaction of electromagnetic radiation, in particular light, with biological tissue is complex and includes the (optical) processes of (multiple) scattering, backscattering, absorption, transmission and (diffuse) reflection. The term "reflect" as used in the context of the present invention is not to be construed as limited to specular reflection but comprises the afore-mentioned types of interaction of electromagnetic radiation, in particular light, with tissue and any combinations thereof.

The term "vital sign" as used in the context of the present invention refers to a physiological parameter of a subject (i.e. a living being) and derivative parameters. In particular, the term "vital sign" comprises blood volume pulse-signal, heart rate (HR) (sometimes also called pulse rate), heart rate variability (pulse rate variability), pulsatility strength, perfusion, perfusion indicator, perfusion variability, Traube Hering Mayer waves, respiratory rate (RR), skin temperature, blood pressure, a concentration of a substance in blood and/or tissue, such as (arterial) blood oxygen saturation or glucose level. Furthermore, "vital sign" generally includes health indications obtained from the shape of the PPG signal (e.g. shape may say something about partial arterial blockage (e.g. shape obtained from PPG signals of the hand gets more sinusoidal when applying a blood-pressure cuff on the arm), or about the skin thickness (e.g. a PPG signal from the face is different than from the hand), or maybe even about the temperature, etc.).

The term "vital sign information" as used in the context of the present invention comprises the one or more measured vital signs as defined above. Furthermore, it comprises data referring to a physiological parameter, corresponding waveform traces or data referring to a physiological parameter of a time that can serve for subsequent analysis.

For obtaining a vital sign information signal of the subject the data signals of skin pixel areas within the skin area are evaluated. Here, a "skin pixel area" means an area comprising one skin pixel or a group of adjacent skin pixels, i.e. a data signal may be derived for a single pixel or a group of skin pixels.

In an embodiment the system further comprises a control unit for controlling said first light source, said second light source and/or said detection unit to synchronize two or more of them with each other. For instance, the second light source may be controlled with the first light source and the detection unit such that only in each second sample of the detection signal transmitted light is measured, but each sample of the detection signal measures reflected light. This improves robustness of the finally obtained vital signs.

This embodiment of time sequential wavelength is applicable for two scenarios. First, when the system needs to decouple the contribution of the second light source from the contribution of an ambient light in the same range. Thus, for each wavelength, the detection unit will measure at even frames the mixture of the dedicated light and ambient light, and on odd frames the contribution of an ambient light only. By taking the difference between two adjacent frames (assuming the frame rate is sufficiently high), the contribution of only the second light source is measured. Second, this embodiment is useful also for another scenario, when a monochrome camera sensor is used as detection unit, which does not have optical filters to differentiate contributions from light sources at different wavelengths. In this scenario the proposed system simulates the behavior of a time sequential multispectral camera.

In another embodiment the system further comprises a control unit for controlling said first light source to emit light in said first wavelength range and/or said second light source to emit light in said second wavelength range. Generally, the first and/or second light source may be designed to emit light at only the first or second wavelength range, respectively. It is, however, preferred to control said wavelength ranges. This, for instance, enables using a single light source (e.g. as second light source) for alternately switching between different wavelength ranges (e.g. between red and infrared wavelength ranges or between two different infrared wavelength ranges).

In another embodiment said detection unit comprises one or more optical filters for filtering light at one or more wavelength ranges out of the detected light. This further improves the accuracy and reliability of obtained vital signs.

Still further, in an embodiment the system further comprises a control unit for controlling said second light source to emit light in said one or more wavelength ranges, in particular in the red and infrared wavelength range, continuously. This is particularly preferred, if the detection unit comprises one or more filters.

The analysis unit is preferably configured for combining said PPG signals for at least two different wavelength ranges and deriving a desired vital sign from the combined PPG signals. There are different options for combining the PPG signals. For instance, an average may be combined of several PPG signals, or PPG signals in the red and infrared wavelength range are used to obtain the oxygen saturation of arterial blood in the generally known manner.

Advantageously, said detection unit is a camera for acquiring a set of image frames of the region of interest. From said image frames the PPG signals can then be extracted. Depending on the desired application, in particular the desired vital signs, a monochrome camera, an RGB camera or a special camera (e.g. optimized for acquisition of certain wavelength ranges) may be used.

The proposed system preferably comprises a first and second light source, e.g. dedicated lamps, for emitting the desired light. Preferably, one or more LEDs are used for this purpose.

The second light source is preferably configured for being arranged behind an ear, similar to a hearing aid to transmit light through the outer part of the ear, or on one side of a hand of a person to transmit light through the hand.

In another embodiment the system further comprises a neonatal intensive care unit (NICU) having a bottom layer, in particular a mattress, wherein said second light source comprises a plurality of light elements, in particular LEDs, arranged in said bottom layer. This enables to unobtrusively and continuously obtaining vital signs of a neonate in a reliable manner.

Preferably, said control input is configured for wirelessly receiving control information.

### BRIEF DESCRIPTION OF THE DRAWINGS

These and other aspects of the invention will be apparent from and elucidated with reference to the embodiments described hereinafter. In the following drawings
Fig. 1 shows a schematic diagram of a system according to the present invention including a device,
Fig. 2 shows schematic diagram of a device,
Fig. 3 shows a graph illustrating the selection of wavelengths of a dedicated illumination for SPO2 measurement,
Fig. 4 shows a diagram illustrating a first example of the alignment of image frame detection with illumination,
Fig. 5 shows a diagram illustrating a second example of the alignment of image frame detection with illumination,
Fig. 6 shows a schematic diagram of another embodiment of a system according to the present invention, and
Fig. 7 shows a schematic diagram of an embodiment of a wearable light source device.

### DETAILED DESCRIPTION OF THE INVENTION

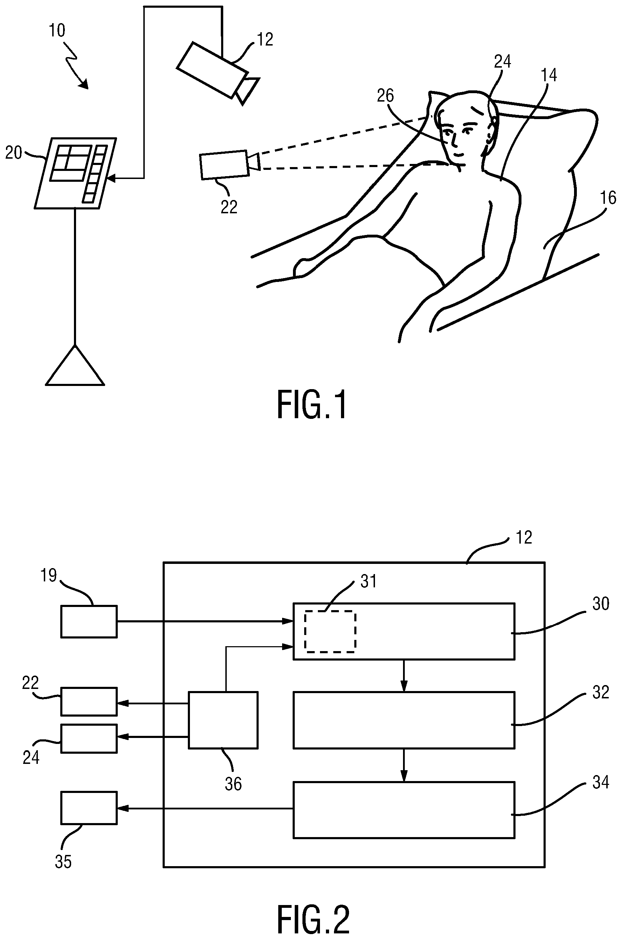

Fig. 1 shows a schematic diagram of a an embodiment of a system 10 according to the present invention including a device 12 for obtaining vital signs of a subject 14. The subject 14, in this example a patient, lies in a bed 16, e.g. in a hospital or other healthcare facility, but may also be a neonate or premature infant, e.g. lying in an incubator, or person at home or in a different environment. Image frames of the subject 14 are captured by means of a camera (also referred to as detection unit or as camera-based or remote PPG sensor) including a suitable photosensor. The camera forwards the recorded image frames to processing means of the device 12, where the image frames will be process as explained in more detail below. The device 12 is further connected to an interface 20 for displaying the determined information and/or for providing medical personnel with an interface to change settings of the device 12 and/or other elements of the system 10. Such an interface 20 may comprise different displays, buttons, touchscreens, keyboards or other human machine interface means.

The image frames captured by the camera may particularly correspond to a video sequence captured by means of an analog or digital photosensor, e.g. in a (digital) camera. Such a camera usually includes a photosensor, such as a CMOS or CCD sensor, which may also operate in a specific spectral range (visible, IR) or provide information for different spectral ranges. The camera may provide an analog or digital signal. The image frames include a plurality of image pixels having associated pixel values. Particularly, the image frames include pixels representing light intensity values captured with different photosensitive elements of a photosensor. These photosensitive elements may be sensitive in a specific spectral range (i.e. representing a specific color). The image frames include at least some image pixels being representative of a skin portion of the subject. Thereby, an image pixel may correspond to one photosensitive element of a photo-detector and its (analog or digital) output or may be determined based on a combination (e.g. through binning) of a plurality of the photosensitive elements.

The system 10 further comprises two light sources 22, 24. A first light source 22, such as a lamp, is provided for illuminating a region of interest 26, such as the skin of the patient's face (e.g. outer part of the ear) with light in a first wavelength range to obtain reflected light from said region of interest 26 which is detected by the camera. A second light source 24, such as an LED or other (preferably miniature) light source, is provided for transmitting light through at least part of the region of interest 26, in particular the outer part of the ear, in a second wavelength range to obtain transmitted light.

The first light source 22 emits light in at least a first wavelength range, e.g. in the wavelength range, which includes green light, and the second light source 24 emits light in at least a second wavelength range different from the first wavelength range, e.g. in the wavelength range of red and/or infrared light. The amplitude of green PPG pulsatility in reflective mode is much larger than red or IR PPG pulsatility in reflective mode. Pulsatility of red and IR PPG pulsatility in transmissive mode is larger than in the reflective mode. Therefore, this embodiment benefits from the combination of large green PPG pulsatility in reflective mode and large PPG pulsatility of IR or red in transmissive mode. However, the present invention not limited only to this distribution of wavelengths between reflective and transmissive modes.

In another embodiment, not according to the invention, no dedicated first light source is provided, but ambient light is used for illumination of the subject in the reflective mode. From the reflective light only light in the desired wavelength range (e.g. green light) is detected and/or evaluated. Thus, also in this embodiment the light portion that is evaluated from the reflective light and the light portion that is evaluated from the transmitted light are in different wavelength ranges.

The transmission of light by the second light source 24 and the illumination of the region of interest with light by the first light source are at least temporarily simultaneous. For instance, the first light source 22 may continuously illuminate the region of interest 26, while the second light source 24 may transmit light through the ear periodically only at certain time intervals, as will be explained in more detail below.

A system 10 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, or the like. The uni- or bidirectional communication between the device 12, the light sources 22, 24 and the interface 20 may work via a wireless or wired communication interface, whereby it is to be noted that one or both of the light sources 22, 24 may also be configured to operate stand-alone and without communication with the device 12.

Fig. 2 shows a more detailed schematic illustration of an embodiment of the device 12. The device 12 comprises a detection unit 30, e.g. a camera, for contactless detection of light in at least two different wavelength ranges from a region of interest of a subject based on a first light portion reflected from said region of interest in response to illumination by a first light source 22 in a first wavelength range and a second light portion transmitted through said region of interest in response to illumination by a second light source 24 in a second wavelength range different from said first wavelength range. Further, a processing unit 32, e.g. a processor, is provided for deriving PPG signals from the detected light 19 (comprising the first and second light portions) for said at least two different wavelength ranges. The way to obtain PPG signals from detected light, e.g. from images of a region of interest, is generally known in the art, e.g. from the above cited documents, and will not be explained in more detail here. Still further, an analysis unit 34 is provided for deriving a desired vital sign 35 from the PPG signals for at least two different wavelength ranges. Also this step is generally known in the art, e.g. from the above cited documents, and will not be explained in more detail here.

Preferably, the device 12 further comprises a control unit 36 for controlling said first light source 22, said second light source 24 and/or said detection unit 30 to synchronize two or more of them with each other. The control unit 36 may further be configured to control said first light source 22 to emit light in said first wavelength range and/or said second light source 24 to emit light in said second wavelength range, for instance to exclusively or alternately emit light in the red or infrared wavelength range.

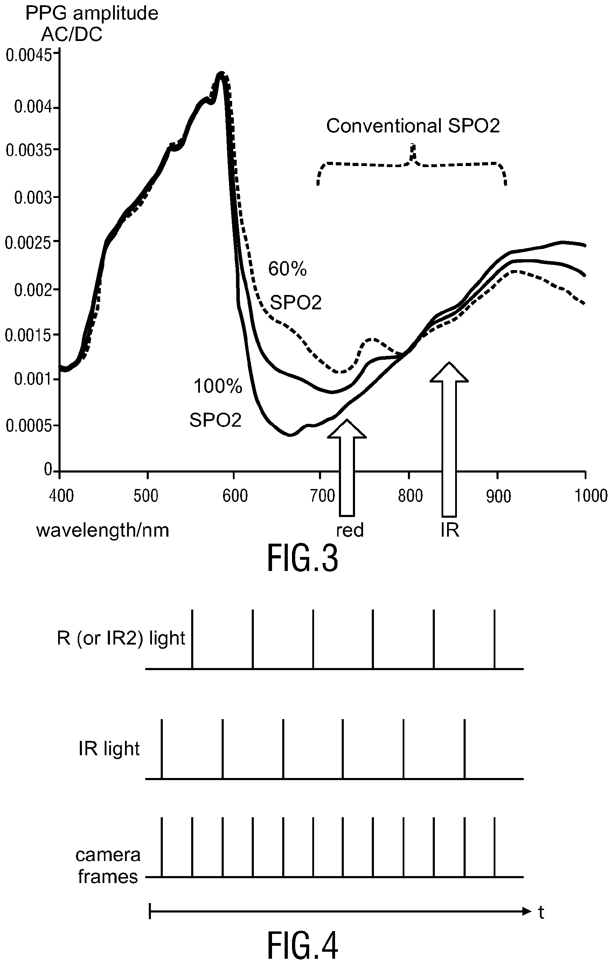

This is illustrated in the diagram shown in Fig. 3, which illustrates an exemplary, non-limiting selection of wavelengths of a dedicated illumination for SPO2 measurement, particularly the dependency of PPG amplitude (AC/DC) for oxygenated (100% SpO2) and low oxygenated (60% SpO2) arterial blood. As shown, the second light source 24 can emit a light with at least one narrow spectrum band in the range > 700 nm for extraction of a heart rate signal. In case SpO2 should be measured, the second light source 24 allows emitting of light in at least two wavelength ranges: 650nm-750nm (red light) and > 800nm (infrared light). The light can be emitted by the second light source 24 either sequentially in time or simultaneously. The wavelengths emitted by the second light source 24 are selected according to the graph shown in Fig. 3.

There are a number of further embodiments, which are based on different combinations of detection unit and light sources and/or different control schemes.

In a rather simple embodiment the system 10 includes one monochrome camera as detection unit and a second light source emitting light in the IR range at one wavelength with a narrow band. In this case the first light is ambient light (at least its visible part of the spectrum). In this case the system measures the combination of PPG pulsatility generated by reflected ambient light and PPG pulsatility generated by transmitted IR from a dedicated light source. That can be actually a main, simplest embodiment. In this embodiment no dedicated first light source is provided, but ambient light (e.g. sun light and/or room light) is used for illumination of the subject in the reflective mode, and from the reflective light only light in the desired wavelength range (e.g. green light) is detected and/or evaluated, for instance by use of filters as mentioned above.

In another embodiment the system comprises one monochrome camera and a second light source emitting light in a red and IR range with at least two wavelengths (preferably with a narrow band) sequentially in time. Frame capturing of a camera is synchronized in time with the second light source in a way that adjacent image frames are captured at different wavelengths of the second light source, as depicted on Fig. 4. Fig. 4 shows a time diagram of the light emission by the second light source 22 and the alignment of said illumination in the IR and red (or a second infrared, IR2) range with the capturing of image frames by the monochrome camera.

In still another embodiment the system comprises a camera with at least two optical filters 31 (shown as optional element with dotted lines in Fig. 2) in the red and IR range in front of the camera's image sensor, which correspond to the narrow bands of the illumination wavelengths of the second light source 22. In this case the light in at least two wavelength bands is preferably emitted continuously.

In still another embodiment the system comprises a camera, which includes three filters in green, red and IR range in front of the camera's image sensor (filters can be arranged similarly to RGB filters of a conventional color camera). Frame capturing of the camera would be synchronized with the second light source 22 operating in red and IR range. This embodiment allows an acquisition of a PPG signal in transmissive and reflective modes simultaneously. The transmissive mode operates in the red and IR range, while the reflective mode operates in the green, red and IR ranges. In an embodiment a combination of a conventional (e.g. finger pulse) sensor of transmissive PPG, which operates at red and IR wavelengths sequentially, and a reflective green PPG. For the reflective mode there can be either ambient or dedicated light, which contains green in the spectrum. For the transmissive mode there will be dedicated light with a spectrum of 650-1000nm.

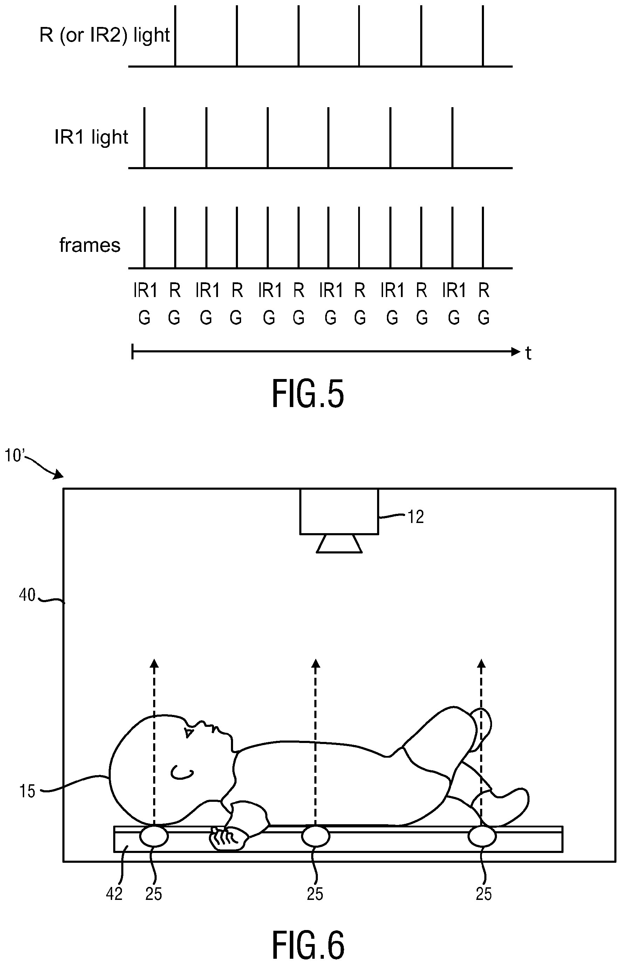

Fig. 5 shows an example of the time synchronization of a camera and the second light source. In this example the second light source emits light with the red (R) (or second infrared, IR2) and first infrared (IR1) wavelengths sequentially in time. The light of the first light is not explicitly shown, but this may be ambient light that is present all the time and covers the whole visible spectrum including green light. That allows improvement of the robustness of the PPG signal acquisition compared to the use of ambient illumination (in the range of red, IR1, IR2), by comparing the corresponding sensor data for each of the red (or IR2) and IR1 wavelengths with and without second illumination from the second light source. The (motion compensated) difference between adjacent frames for each of red (or IR2) and IR1 channels provides an estimation of the second light portion only (without the contribution of ambient illumination). Hereby, motion compensation is e.g. achieved by processing pixels along estimated motion vectors, like in any motion compensation video processing algorithm.

Moreover, sequential switching of the second light source on and off allows an acquisition of reflective PPG, when a dedicated illumination is off. Thus, the system in this embodiment combines a transmissive PPG in channels R (or IR2) and IR1, with reflective PPG in channels green (G), red (or IR2) and IR1. In another embodiment, reflective light in other wavelength ranges, such as the blue and green wavelength range, and transmissive light in the reflective wavelength range may be evaluated if necessary and/or favorable for the respective application and derivation of a desired vital sign.

There are many other embodiments for combination of the transmissive PPG with a dedicated illumination and reflective PPG with ambient illumination or illumination from another dedicated light source.

A preferred embodiment uses a second light source for dedicated illumination that is placed behind a subject's ear (e.g. like a hearing aid), while a camera is located in front of the subject's face. Frame capturing of this camera is synchronized with the second light source, thus acquiring both transmissive PPG signal (with a light emitted from the second light source) and reflective PPG signal (with ambient light or light from a first light source).

Another embodiment of proposed system 10' is depicted in Fig. 6. In this embodiment the second light source comprises a plurality (here 3) light source elements 25, e.g. LEDs or lamps, which are embedded in the mattress 42 of an NICU 40, while a device 12 including a camera is positioned above a baby 15 and acquires both transmissive and reflective light, from which the PPG signals for the derivation of one or more vital signs are obtained as explained above.

It should be noted that the elements of the device 12 may not necessarily combined into a single entity or housing, but may also be arranged in a distributed manner. For instance, the processing unit 32 and the analysis unit 34 (and also the control unit 36) may also be arranged separate from the camera 30, e.g. within the interface 20 or in a separate computer.

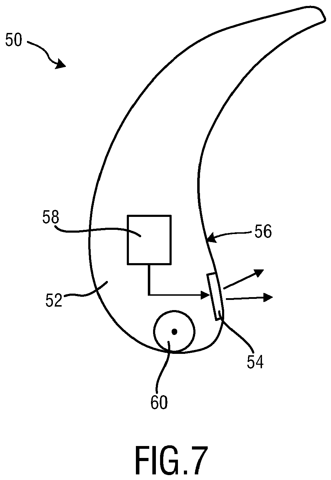

Fig. 7 shows a schematic diagram of an embodiment of a wearable light source device 50. Said wearable light source device 50 is preferably used as second light source 24 shown in Fig. 1.

The wearable light source device 50 comprises a holder 52 for arranging the wearable light source device to a portion of a subject's body, in this embodiment behind a person's ear, in which case the holder has e.g. the form of a hearing aid. A light source 54, e.g. an LED, for emitting light is mounted in or at the holder 52 such that the emitted light is transmitted through a region of interest of the subject for illuminating the region of interest to obtain transmitted light. In this embodiment the light source 54 is arranged at the surface 56 of the holder 52 that faces the rear side of the ear lobe so that the light is transmitted through the ear lobe and the transmitted light can be seen and captured from the front side of the ear lobe.

A control input 58 is provided for receiving control information, e.g. from the control unit 36 shown in Fig. 2, for controlling the light source 54 in a way as described above. For instance, in an embodiment the light source is controlled such that frame capturing of the camera 12 is synchronized in time with the light source 54 in a way that adjacent image frames are captured at different wavelengths of the light source 54. Preferably, said control input 58 is configured for wirelessly receiving control information, e.g. via Bluetooth, WLAN, Zigbee, etc.

Further, preferably a power source 60, such as a battery is provided in order to avoid any cabling and ensure self-sustaining operation of the wearable light source device 50.

In summary, the present invention provides substantial advantages over the existing SpO2 measurement methods using a clamping sensor (clamped to the finger or earlobe) or a camera (using rPPG). In particular, an SpO2 clamping device is sensitive to motion and disturbances caused by ambient light. The additional (second) light source provided according to the present invention may be a battery supplied LED which is placed behind the ear. It may be wireless device and does not need to be "clamped". Further, the quality of the SpO2 measurement (in terms of SNR) of the SpO2 camera measurement is enhanced.

By way of example, the present invention can be applied in the field of health care, e.g. unobtrusive remote patient monitoring, general surveillances, security monitoring and so-called lifestyle environments, such as fitness equipment, or the like. Applications may include monitoring of oxygen saturation (pulse oximetry), heart rate, blood pressure, cardiac output, changes of blood perfusion, assessment of autonomic functions, and detection of peripheral vascular diseases. The present invention can particularly be used for rapid and reliable pulse detection of a critical patient, for instance during automated CPR (cardiopulmonary resuscitation). The system can be used for monitoring of vital signs of neonates as well. In general, the present invention allows both spot-check and continuous monitoring.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. System (10, 10') for determining vital signs of a subject comprising:
a device (12) for determining vital signs of a subject,
a first light source (22) arranged for illuminating a front side of a region of interest of the subject with light in at least a first wavelength range to obtain reflected light that is reflected from the region of interest,
a second light source (24, 25) arranged for transmitting light in at least a second wavelength range different from the first wavelength range from a rear side of the region of interest through the region of interest to obtain transmitted light that is transmitted through the region of interest,
wherein the first and and second light sources are configured to illuminate the region of interest and to transmit light through the region of interest at least temporarily simultaneous, and
wherein the device comprises:
- a single detection unit (30) arranged for contactless detection of light in at least two different wavelength ranges from the region of interest of the subject and for contactless detection of the reflected light and the transmitted light from the front side of the region of interest of the subject, wherein said detection unit (30) is configured to detect a first light portion in the first wavelength range from the reflected light and to detect a second light portion in the second wavelength range from the transmitted light, wherein said detection unit (30) is configured to detect said first light portion and said second light portion simultaneously,
- a processing unit (32) for deriving plethysmography, PPG, signals from the detected light for said at least two different wavelength ranges, and
- an analysis unit (34) for deriving a vital sign from the PPG signals for at least two different wavelength ranges.

2. System as claimed in claim 1,
further comprising a control unit (36) for controlling said first light source (22), said second light source (24) and/or said detection unit (30) to synchronize two or more of them with each other.

3. System as claimed in claim 1,
further comprising a control unit (36) for controlling said first light source to emit light in said first wavelength range and/or said second light source (24) to emit light in said second wavelength range.

4. System as claimed in claim 3,
wherein said control unit (36) is configured to control said second light source (24) to exclusively or alternately emit light in the red or infrared wavelength range which represents the second wavelength range.

5. System as claimed in claim 1,
wherein said detection unit (30) comprises one or more optical filters (31) for filtering light at said first and second wavelength ranges out of the detected light.

6. System as claimed in claim 1,
further comprising a control unit (36) for controlling said second light source (24) to emit light in said second wavelength range, in particular in the red and infrared wavelength range which represents the second wavelength range, continuously.

7. System as claimed in claim 1,
wherein said analysis unit (34) is configured for combining said PPG signals for at least two different wavelength ranges and deriving the vital sign from the combined PPG signals.

8. System as claimed in claim 1,
wherein said detection unit (30) is a camera for acquiring a set of image frames of the region of interest.

9. System as claimed in claim 1,
wherein said second light source (24) is configured for being arranged behind an ear or on one side of a hand of a person.

10. System as claimed in claim 1,
further comprising a neonatal intensive care unit (40) having a bottom layer, in particular a mattress (42), wherein said second light source comprises a plurality of light elements (25), in particular LEDs, arranged in said bottom layer.

11. System as claimed in claim 1,
further comprising a wearable light source device (50) comprising:
- a holder (52) for arranging the wearable light source device to a portion of a subject's body, in particular behind an ear or on one side of a hand of a person, and
- a control input (58) for receiving control information for controlling the second light source (54),
wherein the second light source (54) is mounted in or at the holder such that the emitted light is transmitted through the region of interest of the subject.

12. System as claimed in claim 11,
wherein said control input (58) is configured for wirelessly receiving control information.

13. Method for determining vital signs of a subject comprising:
- illuminating, by a first light source (22), a front side of a region of interest of the subject with light in at least a first wavelength range to obtain reflected light that is reflected from the region of interest,
- transmitting, by a second light source (24, 25), light in at least a second wavelength range different from the first wavelength range from a rear side of the region of interest through the region of interest to obtain transmitted light that is transmitted through the region of interest, wherein the illumination of the region of interest and the transmission of light through the region of interest are at least temporarily simultaneous,
- contactlessly detecting, by a single detection unit (30), light in at least two different wavelength ranges from the region of interest of the subject and contactlessly detecting the reflected light and the transmitted light from the front side of the region of interest of the subject, wherein a first light portion is detected in the first wavelength range from the reflected light and a second light portion is detected in the second wavelength range from the transmitted light, wherein said first light portion and said second light portion are detected simultaneously,
- deriving, by a processing unit (32), plethysmography, PPG, signals from the detected light for said at least two different wavelength ranges, and
- deriving, by an analysis unit (34), a vital sign from the PPG signals for at least two different wavelength ranges.

## Patentansprüche

1. System (10, 10') zur Bestimmung der Vitalparameter eines Patienten, das Folgendes umfasst:
ein Gerät (12) zur Bestimmung der Lebenszeichen eines Patienten,
eine erste Lichtquelle (22), die so angeordnet ist, dass sie eine Vorderseite eines betreffenden Bereichs des Patienten mit Licht in mindestens einem ersten Wellenlängenbereich zu beleuchten, um reflektiertes Licht zu erhalten, das von dem betreffenden Bereich reflektiert wird,
eine zweite Lichtquelle (24, 25), die so angeordnet ist, dass sie Licht in mindestens einem zweiten Wellenlängenbereich, der sich von dem ersten Wellenlängenbereich unterscheidet, von einer Rückseite des betreffenden Bereichs durch den betreffenden Bereich sendet,
um übertragenes Licht zu erhalten, das durch den betreffenden Bereich gesendet wird, wobei die erste und die zweite Lichtquelle so konfiguriert sind, dass sie den betreffenden Bereich beleuchten und zumindest zeitweise gleichzeitig Licht durch den betreffenden Bereich übertragen, und
wobei das Gerät Folgendes umfasst:
- eine einzelne Erfassungseinheit (30), die zur kontaktlosen Erfassung von Licht in mindestens zwei verschiedenen Wellenlängenbereichen aus dem betreffenden Bereich des Patienten und zur kontaktlosen Erfassung des reflektierten Lichts und des übertragenen Lichts von der Vorderseite des betreffenden Bereichs des Patienten angeordnet ist, wobei die Erfassungseinheit (30) so konfiguriert ist, dass sie einen ersten Lichtanteil in dem ersten Wellenlängenbereich aus dem reflektierten Licht erfasst und einen zweiten Lichtanteil in dem zweiten Wellenlängenbereich aus dem übertragenen Licht erfasst, wobei die Erfassungseinheit (30) so konfiguriert ist, dass sie den ersten Lichtanteil und den zweiten Lichtanteil gleichzeitig erfasst,
- eine Verarbeitungseinheit (32) zum Ableiten von Plethysmographiesignalen, PPG, aus dem detektierten Lichts für die mindestens zwei verschiedenen Wellenlängenbereiche, und
- eine Analyseeinheit (34) zur Ableitung eines Vitalzeichens aus den PPG-Signalen für mindestens zwei verschiedene Wellenlängenbereiche.

2. System nach Anspruch 1,
ferner mit einer Steuereinheit (36) zum Steuern der ersten Lichtquelle (22), der zweiten Lichtquelle (24) und/oder der Detektionseinheit (30), um zwei oder mehrere von ihnen miteinander zu synchronisieren.

3. System nach Anspruch 1,
ferner mit einer Steuereinheit (36) zum Steuern der ersten Lichtquelle, um Licht im Wellenlängenbereich und/oder der zweiten Lichtquelle (24) zur Emission von Licht im zweiten Wellenlängenbereich

4. System nach Anspruch 3,
wobei die Steuereinheit (36) so konfiguriert ist, dass sie die zweite Lichtquelle (24) so steuert, dass sie ausschließlich oder abwechselnd Licht in dem roten oder infraroten Wellenlängenbereich emittiert, der den zweiten Wellenlängenbereich darstellt.

5. System nach Anspruch 1,
wobei die Erfassungseinheit (30) einen oder mehrere optische Filter (31) zum Herausfiltern von Licht im ersten und zweiten Wellenlängenbereich aus dem detektierten Licht umfasst.

6. System nach Anspruch 1,
ferner mit einer Steuereinheit (36) zum Steuern der genannten zweiten Lichtquelle (24) zum kontinuierlichen Emittieren von Licht in dem zweiten Wellenlängenbereich, insbesondere im roten und infraroten Wellenlängenbereich der den zweiten Wellenlängenbereich darstellt.

7. System nach Anspruch 1,
wobei die genannte Analyseeinheit (34) so konfiguriert ist, dass sie die genannten PPG-Signale für mindestens zwei verschiedene Wellenlängenbereichen kombiniert und das Vitalzeichen aus den kombinierten PPG-Signalen ableitet.

8. System nach Anspruch 1,
wobei die Erfassungseinheit (30) eine Kamera zum Erfassen eines Satzes von Bildrahmen des betreffenden Bereichs ist.

9. System nach Anspruch 1,
wobei die zweite Lichtquelle (24) so konfiguriert ist, dass sie hinter einem Ohr oder auf einer Seite einer Hand einer Person angebracht wird.

10. System nach Anspruch 1,
das ferner eine neonatale Intensivstation (40) mit einer Unterlage, insbesondere einer Matratze (42), umfasst, wobei die zweite Lichtquelle eine Vielzahl von Lichtelementen (25), insbesondere LEDs, umfasst, die in der Unterlage angeordnet sind.

11. System nach Anspruch 1,
das ferner ein tragbares Lichtquellengerät (50) umfasst:
- eine Halterung (52) zum Anbringen des tragbaren Geräts an einem Körperteil eines Patienten, insbesondere hinter einem Ohr oder an einer Seite einer Hand einer Person, und
- einen Steuereingang (58) zum Empfangen von Steuerinformationen zum Steuern der zweiten Lichtquelle (54),
wobei die zweite Lichtquelle (54) in oder an der Halterung so angebracht ist, dass das emittierte Licht durch den betreffenden Bereich des Patienten übertragen wird.

12. System nach Anspruch 11,
wobei der Steuereingang (58) für den drahtlosen Empfang von Steuerinformationen konfiguriert ist.

13. Verfahren zur Bestimmung der Lebenszeichen eines Subjekts, umfassend:
- Beleuchten einer Vorderseite eines betreffenden Bereichs des Patienten durch eine erste Lichtquelle (22) mit Licht in mindestens einem ersten Wellenlängenbereich, um reflektiertes Licht zu erhalten, das von dem betreffenden Bereich reflektiert wird,
- Übertragen von Licht in mindestens einem zweiten Wellenlängenbereich, der sich von dem ersten Wellenlängenbereich unterscheidet, durch eine zweite Lichtquelle (24, 25) von einer Rückseite des betreffenden Bereichs durch den betreffenden Bereich hindurch übertragen wird, um übertragenes Licht zu erhalten, wobei die Beleuchtung des betreffenden Bereichs und die Übertragung von Licht durch den betreffenden Bereich hindurch zumindest zeitweise simultan sind,
- Kontaktlose Erfassung von Licht in mindestens zwei verschiedenen Wellenlängenbereichen aus dem betreffenden Bereich des Patienten durch eine einzelne Erfassungseinheit (30) und kontaktloses Erfassen des reflektierten Lichts und des übertragenen Lichts von der Vorderseite des betreffenden Bereichs des Patienten, wobei ein erster Lichtanteil dem reflektierten Licht im ersten Wellenlängenbereich erfasst wird und ein zweiter Lichtanteil im zweiten Wellenlängenbereich aus dem übertragenen Licht erfasst wird, wobei der erste Lichtanteil und der zweite Lichtanteil gleichzeitig erfasst werden,
- Ableitung von Plethysmographiesignalen (PPG) durch eine Verarbeitungseinheit (32) aus dem detektierten Licht für die mindestens zwei verschiedenen Wellenlängenbereiche, und
- Ableitung eines Vitalzeichens durch eine Analyseeinheit (34) aus den PPG-Signalen für mindestens zwei verschiedene Wellenlängenbereiche.

## Revendications

1. Un système (10, 10') pour déterminer des signes vitaux d'un sujet comprend:
un dispositif (12) pour déterminer les signes vitaux chez un sujet,
une première source de lumière (22) prévue pour éclairer une face avant d'une région d'intérêt du sujet avec une lumière dans au moins une première plage de longueur d'onde pour obtenir une lumière réfléchie qui est réfléchie à partir de la région d'intérêt,
une seconde source de lumière (24, 25) prévue pour transmettre la lumière dans au moins une seconde plage de longueur d'onde différente à partir d'une première plage de longueur d'onde à l'arrière de la région d'intérêt à travers la région d'intérêt pour obtenir une lumière transmise qui est transmise à travers la région d'intérêt,
où la première et seconde lumière source sont configurées pour éclairer la région d'intérêt et pour transmettre la lumière à travers la région d'intérêt au moins de manière simultanée, et
où le dispositif comprend:
- une seule unité de détection (30) prévue pour la détection sans contact d'une lumière dans au moins deux plages de longueurs d'onde différentes à partir de la région d'intérêt du sujet et pour la détection sans contact de la lumière réfléchie et de la lumière transmise à partir de l'arrière de la région d'intérêt du sujet, où ladite unité de détection (30) est configurée pour détecter une première partie de lumière dans la première plage de longueur d'onde à partir de la lumière réfléchie et pour détecter la lumière et pour détecter une seconde partie de lumière dans une seconde plage de longueurs d'onde à partir de la lumière transmise, où ladite unité de détection (30) est configurée pour détecter ladite première partie de lumière et ladite seconde partie de lumière simultanément,
- une unité de traitement (32), pour obtenir des signaux de photopléthysmographie PPG, à partir de la lumière détectée pour au moins deux longueurs d'onde différentes, et
- une unité d'analyse (34) pour obtenir un signe vital à partir des signaux PPG pour au moins deux plages de longueurs d'onde différentes.

2. Un système comme revendiqué dans la revendication 1, comprend également une unité de contrôle (36) pour contrôler ladite première source de lumière (22), ladite seconde source de lumière (24) et/ou ladite unité de détection (30) pour synchroniser deux ou plusieurs unités l'une avec l'autre.

3. Un système comme revendiqué dans la revendication 1, comprend également une unité de contrôle (36) pour contrôler ladite première source de lumière pour émettre une lumière dans ladite première longueur d'onde et/ou ladite seconde source de lumière (24) pour émettre une lumière dans la seconde plage de longueur d'onde.

4. Un système comme revendiqué dans la revendication 3, où l'unité de commande (36) est configurée pour contrôler ladite seconde de source de lumière (24) pour émettre exclusivement ou alternativement de la lumière dans la plage de longueur d'onde rouge ou infrarouge qui représente la seconde plage de longueur d'onde.

5. Un système comme revendiqué dans la revendication 1, où ladite unité de détection (30) comprend un ou plusieurs filtres optiques (31) pour filtrer la lumière desdites premières et secondes longueurs d'onde situées en dehors de la lumière détectée.

6. Un système comme revendiqué dans la revendication 1, comprend également une unité de contrôle (36) pour contrôler ladite seconde source de lumière (24) pour émettre la lumière dans ladite seconde plage de longueurs d'onde, en particulier dans les plages de longueurs d'onde rouge et infrarouge qui représente la seconde plage de longueur d'onde, en continu.

7. Un système comme revendiqué dans la revendication 1, où ladite unité d'analyse (34) est configurée pour combiner lesdits signaux PPG pour au moins deux longueurs d'onde différentes et obtenir des signes vitaux à partir de signaux PPG combinés.

8. Un système comme revendiqué dans la revendication 1, où ladite unité de détection (30) est un appareil photo pour obtenir un ensemble d'images de la région d'intérêt.

9. Un système comme revendiqué dans la revendication 1, où ladite seconde source de lumière (24) est configurée pour être disposée derrière une oreille sur le côté d'une main d'une personne.

10. Un système comme revendiqué dans la revendication 1, comprend une unité néonatale de soins intensifs (40) possédant une couche inférieure, en particulier un matelas (42), où la seconde source de lumière comprend une pluralité d'éléments de lumière (25), en particulier des LEDs, disposées dans la couche inférieure.

11. Un système comme revendiqué dans la revendication 1, comprend un dispositif de source de lumière portable (50) comprenant:
- un support (52) pour disposer le dispositif de source lumineuse portable vers une partie d'un corps d'un sujet, en particulier derrière une oreille ou un côté de la main d'une personne, et
- une entrée de commande (58) pour recevoir des informations de commande pour contrôler une seconde source de lumière (54),
où la seconde source de lumière (54) est montée dans ou
sur le support de sorte que la lumière émise est transmise à travers la région d'intérêt du sujet.

12. Un système comme revendiqué dans la revendication 11, où ladite unité de contrôle (58) est configurée pour recevoir sans fil des informations de contrôle.

13. Une méthode pour déterminer les signes vitaux d'un sujet comprenant:
- l'éclairage, par une première source de lumière (22), une face avant d'une région d'intérêt du sujet avec la lumière dans au moins une première plage de longueur d'onde pour obtenir une lumière réfléchie qui est reflétée à partir de la région d'intérêt,
- la transmission par une seconde source de lumière (24, 25), dans au moins une seconde plage de longueur d'onde différente à partir de la première plage de longueur d'onde d'une face arrière de la région d'intérêt à travers la région d'intérêt pour obtenir la lumière transmise qui est transmise à travers la région d'intérêt, où l'éclairage de la région d'intérêt et la transmission de lumière à travers la région d'intérêt sont au moins temporairement simultanés,
- la détection sans contact, par une seule unité de détection (30), la lumière dans au moins deux longueurs d'onde différentes à partir de la région d'intérêt du sujet et la détection sans contact de la lumière réfléchie et la lumière transmise à partir de la face avant de la région d'intérêt du sujet, où une première partie de lumière est détectée dans la première plage de longueur d'onde à partir de la lumière réfléchie et une seconde partie de lumière est détectée dans la seconde plage de longueur d'onde, à partir de la lumière transmise, où ladite première partie de lumière et ladite seconde partie de lumière sont détectées de façon simultanée,
- l'obtention, par une unité de traitement (32), des signaux de photopléthysmographie, PPG à partir de la lumière détectée pour au moins deux longueurs d'onde différentes, et
- l'obtention, par une unité d'analyse (34), d'un signe vital à partir des signaux PPG pour au moins deux longueurs d'onde différentes.
